Europäisches Patentamt

European Patent Office

Office européen des brevets

(11) Publication number: **0 479 502 B1**

## EUROPEAN PATENT SPECIFICATION

(45) Date of publication of patent specification: **15.11.95**  (51) Int. Cl.⁶: **A61B 3/103**

(21) Application number: **91308854.8**

(22) Date of filing: **27.09.91**

(54) Apparatus for measuring ocular refracting power.

| | |
|---|---|
| (30) Priority: **29.09.90 JP 262766/90** | (73) Proprietor: **Kabushiki Kaisha TOPCON** **75-1, Hasunuma-cho** **Itabashi-ku** **Tokyo (JP)** |
| (43) Date of publication of application: **08.04.92 Bulletin 92/15** | |
| (45) Publication of the grant of the patent: **15.11.95 Bulletin 95/46** | (72) Inventor: **Ishikura, Yasuhisa,, c/o Kabushiki** **Kaisha Topcon** **75-1, Hasunuma-cho,** **Itabashi-ku** **Tokyo-to (JP)** **Inventor: Kitao, Ikuo, c/o Kabushiki Kaisha** **Topcon** **75-1, Hasunuma-cho,** **Itabashi-ku** **Tokyo-to (JP)** |
| (84) Designated Contracting States: **CH DE ES FR GB IT LI** | |
| (56) References cited: **EP-A- 0 189 350** **EP-A- 0 373 788** **FR-A- 2 372 617** **US-A- 4 660 946** | |
| **MACHINE DESIGN, vol. 52, no. 12, May 1980, Cleveland, US, page 48, "Eyeglass Prescription via Electronics"** | (74) Representative: **Jennings, Nigel Robin et al** **KILBURN & STRODE** **30 John Street** **London WC1N 2DD (GB)** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

The present invention relates to an apparatus for measuring ocular refracting power and is concerned with such apparatus which includes a light source arranged to emit a light beam along an optical axis and to project an image of the light source onto the retina of the eye to be tested, a light receiving system including a light blocking member for blocking a part of the light beam reflected from the retina of the eye, a photodetector which is placed at a position approximately conjugate with the pupil of the eye and is responsive to the gradient of the light intensity of the light beam incident on it along a line corresponding to a meridian line on the eye and produces a signal representative of the said gradient, and a controller arranged to calculate the ocular refracting power based on the said gradient on the meridian line.

An ocular refracting power measuring system of so-called photo-refraction type is known. In this system, the retina of the eye to be tested is illuminated by stroboscopic light, the light intensity conditions on the pupil of the eye are recorded by a camera, and the ocular refracting power of the eye is calculated from these results.

In a system of so-called photorefraction type, a photograph is taken with an ordinary camera. As a result, the image on the film must be analysed and the ocular refracting power must then be calculated from the results of the analysis. Thus, it is not possible to obtain an accurate result. Furthermore, the calculation can only be made after developing the film, and this means that it is impossible to obtain the result instantaneously.

The astigmatic condition of the eye is not determined in this known system and this must be done with a quite separate system.

In Japanese Provisional Patent Publication No. 191428/1990, the present applicant has proposed an ocular refracting power measuring system which can measure the ocular refracting power accurately and instantaneously on the basis of the variation of light intensity. In this system, the image of a light source is projected onto the retina of the eye to be tested, and the light beam reflected from the retina is partially blocked by an edge-like light blocking member. The light beam thus blocked is received by a light receiving element, and the ocular refracting power is measured based on the variation of the incident light intensity.

It is the object of the present invention to provide an apparatus for measuring the ocular refracting power of the type referred to above which offers the advantages of the apparatus disclosed in the prior Japanese application referred to above but which can also measure the astigmatic condition of the eye at the same time.

EP-A-0 373 788 discloses an apparatus for measuring the ocular refractive power based on the same principle. It comprises either a linear rotatable or a hexagonal blocking member enabling the determination of astigmatism based on measurements of the refractive power at different angles.

In accordance with the present invention an apparatus for measuring the ocular refracting power of the type referred to above is described by Claim 1. Thus in the apparatus in accordance with the invention the ocular refracting power of the eye is measured on a plurality of meridians and the values of the refracting power are stored and used instantaneously to calculate the astigmatic condition of the eye.

The light blocking member may be in the form of an annular member, the edge of the aperture in which is circular or it may be in the form of a disc whose outer edge is circular. The projector system is preferably so constructed that the emitted light beam is slit-shaped, that is to say of elongate shape. The light beam is rotated about the optical axis between successive measurements on different meridian lines and the fact that the edge of the light blocking member is circular, with the centre of the circle line on the axis of the reflected beam, means that the same proportion of the beam is blocked regardless of the orientation of the beam.

The light source itself may be slit-shaped and is preferably connected to a motor to be rotated about the optical axis. Alternatively, the light source need not be slit-shaped and in this case the projector system may include a diaphragm in which a slit-shaped aperture is formed. In this case the aperture may be connected to a motor to be rotated about the optical axis.

The photodetector may include a planar light-receiving element or sensor. In this event no movement of the light receiving element is necessary between successive measurements of the ocular refracting power on different meridian lines. Alternatively, the photodetector may include a line sensor, that is to say an elongate sensor, and since the length of the line sensor will, in practice, need to be parallel with the meridian line on which the ocular refracting power is being measured it will be necessary to rotate the line sensor between successive measurements of the ocular refracting power on different meridians. In order to ensure that the slit-shaped light beam emitted from the projector system and the line sensor are correctly orientated with respect to one another during each measurement of the ocular refracting power it is preferred that the motors are connected to respective drivers which are connected, e.g. to a common controller, so that the two motors rotate in synchronism.

Further features and details of the invention will be apparent from the following description of certain specific embodiments which is given by way of example with reference to the accompanying diagrammatic drawings, in which:-

Figure 1 shows the basic configuration of one embodiment of the invention;

Figure 2 shows the relationship between the light blocking member and light beam; and

Figures 3, 4 and 5 show the basic configuration of the second, third and fourth embodiments of the invention, respectively.

In the ocular refracting power measuring system disclosed in Japanese Provisional Patent Publication No. 191428/1990, the light beam from the light source is reflected from the retina and is then partially blocked by an edge-like light blocking member. The light beam thus blocked is received by a light receiving element and the ocular refracting power is measured based on the light incident on the light receiving element. When the blocked light beam is received by the light receiving element, the distribution of the light-intensity on the light receiving element is a function of the ocular refracting power and varies along a line on the light receiving element, corresponding to a meridian line on the eye due to the influence of the blocking member. Astigmatism is present due to a difference in the ocular refracting power (diopter) on different meridian lines, that is to say mutually inclined lines on the eye, and the condition of astigmatism is defined by the spherical curvature S, the degree of astigmatism C, and the angle of the astigmatic axis A. The relationship between the diopter $D_\theta$ and the spherical curvature S, the degree of astigmatism C, the angle of astigmatic axis A on the meridian line at an arbitrary angle $\theta$ is given by the following formula:

$$D_\theta = S + C \sin(\theta - A) \qquad (I)$$

Therefore, if the values of the diopter on three different meridian lines of angles $\theta_1$, $\theta_2$ and $\theta_3$ are known, the spherical curvature S, the degree of astigmatism C and the angle of the astigmatic axis A can be obtained. This allows the definition of the condition of astigmatism.

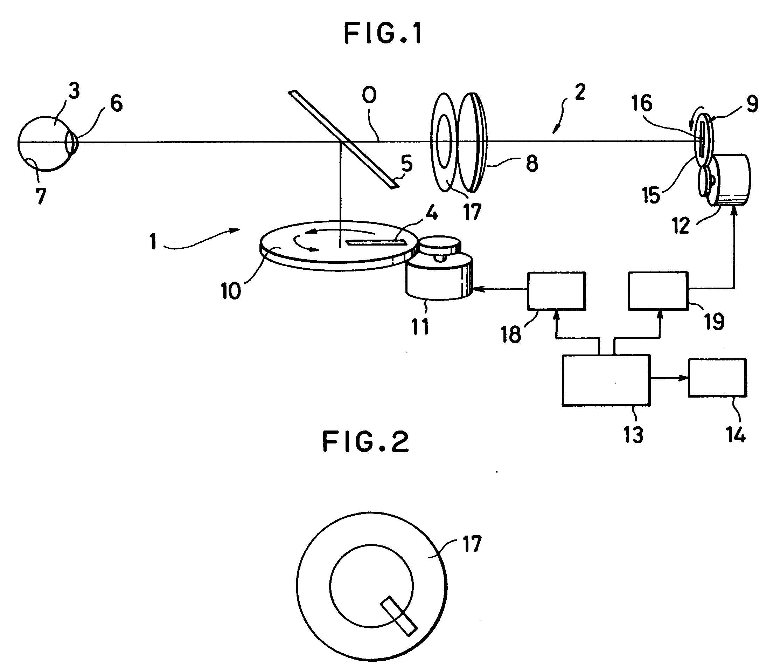

Referring now to Figure 1, 1 represents a projector system for projecting the image of the measuring light source on the retina 7 of the eye to be tested 3, and 2 represents a light receiving system for receiving the light beam reflected from the retina 7. The projector system 1 and the light receiving system 2 are placed to project a light beam into and to receive a light beam from the eye 3 to be tested, respectively.

The projector system 1 comprises a fine slit-shaped measuring light source 4 extending radially on a disk 10, which is arranged to be rotated around the optical axis of the projector system by a motor 11, and a half-mirror or semi-silvered mirror 5 arranged to reflect the light beam from the measuring light source 4 towards the eye to be tested.

Further, the projector system 1 projects a light beam from the measuring light source 4 in such manner that an image of the measuring light source 4 is formed on the retina 7 through the pupil 6.

The light receiving system 2 comprises an objective lens 8 and a photodetector (light receiver) 9. The light beam reflected from the retina 7 is conducted onto the photodetector 9 through the half-mirror 5.

The photodetector 9 comprises a light receiving plate 15 mounted to rotate around the optical axis of the light receiving system and a line sensor 16 mounted on a diameter of the light receiving plate 15. The light receiving plate 15 is connected to be rotated by a motor 12.

The line sensor 16 is disposed in the conjugate position with the pupil 6 of the eye to be tested about the objective lens 8.

Provided in the optical path of the light receiving system is a light blocking member 17 which lies within a plane perpendicular to the optical axis O and at a position where the image of the measuring light source is formed when the ocular refracting power of the eye to be tested 3 is the standard diopter. The light blocking member 17 is thus substantially conjugate with the retina 7 about the pupil 6. The light blocking member 17 is an annular plate, the edge of the aperture in which is circular. The light blocking member 17 blocks a part of the light beam reflected from the retina of the eye to be tested.

The motor 11 for the light source and the motor 12 for the photodetector are driven by motor drivers 18 and 19, and the driving command from a controller 13 is supplied to both of these drivers 18 and 19. The two motors 11 and 12 are thus driven synchronously by the controller 13.

The photodetector 9 is connected to the controller 13, and the signal from the photodetector 9, i.e. the signal produced when light is incident on the line sensor 16 is supplied as an input to the controller 13. The controller 13 memorises the intensity gradient of the light incident on the line sensor 16 and calculates the ocular refracting power therefrom and is arranged also to calculate the astigmatic condition from the ocular refracting powers measured on a plurality of meridian lines and to supply the results to a display unit 14, as appropriate.

The apparatus described above is used as follows:-

A slit-shaped light beam is projected by the fine slit-shaped measuring light source 4 onto the retina of the eye to be tested and a part of the light beam reflected from the retina of the eye to be tested is blocked by means of the light blocking member 12. The reflected light beam, a part of which has been blocked by the light blocking member, is projected on the photodetector 9. The relative angular positions of the measuring light source 4 and the photodetector 9 are controlled in such a manner that the direction of the line sensor 16 of the photodetector 9 concurs with that of the reflected light beam.

The intensity of the light incident on the line sensor 16 varies along its length and the line sensor 16 produces an output signal which is a function of the change in light intensity or light gradient and supplies this output to the controller 13. The line defined by the line sensor along which the light intensity varies corresponds to a meridian on the eye. The degree of variation of the incident light intensity, i.e. the gradient of the distribution of the incident light intensity, represents the ocular refracting power, and the direction of the gradient depends upon whether the ocular refracting power of the eye to be tested is bigger or smaller than the standard diopter. The value of the ocular refracting power is calculated by the controller 13 and then store.

As stated in Japanese Provisional Patent Publication No. 191428/1990, the deviation $\Delta D$ of the diopter is given by:

$$\Delta D = \alpha L D_0 / \mu,$$

where $\alpha$ is the gradient of the distribution of light amount, $D_0$ the standard diopter, $\mu$ the diameter of the pupil, and $L$ the size of the light source. Thus, the diameter of the pupil is needed for the calculation. To eliminate the influence of individual differences in the pupil diameter, it is preferable to install a diaphragm (not shown) of a size smaller than the pupil diameter of any eye to be tested at the conjugated position of the eye to be tested on the projected side and light receiving side, or at least on the projected side.

If the diaphragm is placed only on the projected side, only the data on the site corresponding to the diaphragm on the projected side of the light receiving plate 15 of the photodetector should be processed.

In case it is necessary to compensate the aberration of the eye to be tested by pupil diameter, the compensation value corresponding to each of the pupil diameters may be added. Or, a changeover switch may be used (not shown). In this case a plurality of different compensation values are input into the controller and the appropriate compensation value is selected by means of the changeover switch. Or, only the data relating to a certain proportion of the pupil diameter of the eye to be tested may be used for calculation.

When the measurement in one meridian direction is completed, the motor 11 of the light source and the motor 12 of the photodetector are rotated synchronously through the angle required, and the ocular refracting power along a second meridian direction is measured. This is then repeated and the ocular refracting power on a third meridian line is measured.

In order to measure the gradient of the intensity incident on the line sensor 16 on each of the meridian lines, the disk 10 and the photodetector 14 may be intermittently driven, or they may be synchronously rotated, and the output signals from the line sensor at three predetermined angles may be processed and stored.

By using the results of the measurement of the ocular refracting power on at least 3 meridian lines, the spherical curvature S, the degree of astigmatism C, and the angle of astigmatic axis A can be obtained promptly with the aid of the above equation (I).

Thus it is possible by using the present invention to easily measure the ocular refracting power on an arbitrary number of meridian lines and to measure the astigmatic condition simultaneously with the measurement of the ocular refracting power of the eye to be tested.

The measuring accuracy can be improved by measuring the ocular refracting power at two positions differing by 180° on a meridian line and averaging the results.

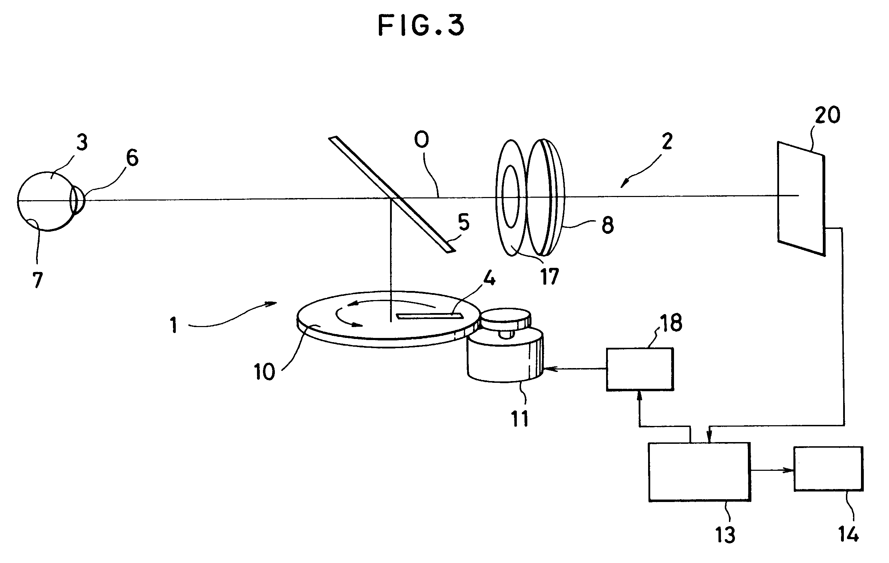

The embodiment of Figure 3 is generally similar to that described above but a planar light receiving element 20 consisting of CCD (charge coupled device) is used instead of the line sensor 16. Such a planar sensor can detect light intensity over a larger area and thus the rotating motor 12 for the photodetector 9 may be omitted.

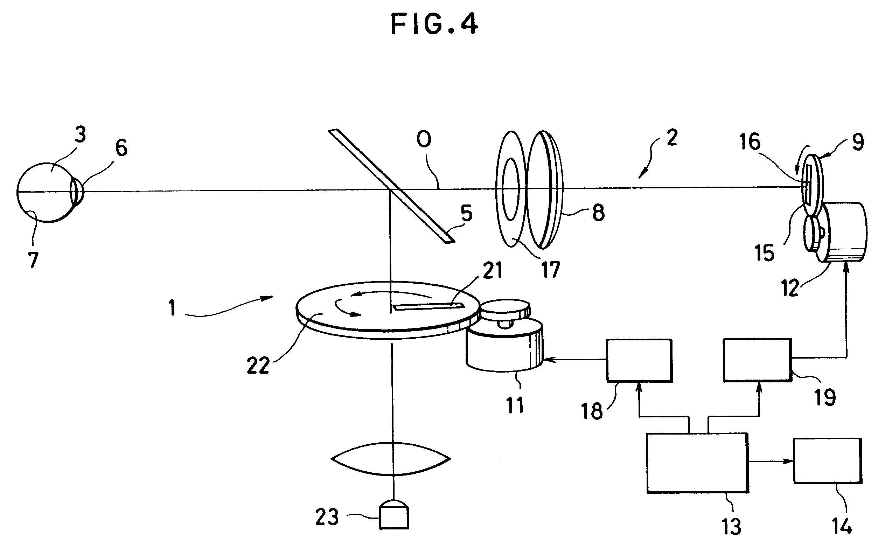

The embodiment of Figure 4 is again generally similar but instead of the slit-shaped light source 4 a diaphragm 22 with a fine radial slit 21 in it is provided at a conjugate position with respect to the eye to be tested 3. Behind the diaphragm is a separate light source 23. The slit 21 ensures that the measuring light beam is slit-shaped and when the ocular refracting power is to be measured on different meridians the light beam is rotated about the axis by rotating the diaphragm by means of the motor 11.

In a further modification which is not illustrated, the diaphragm 22 or disc 10 is not rotatable but is provided with three or more radially extending light sources, on the disc or in slits in the diaphragm. These light sources are actuated sequentially to produce an effective rotation of the light beam about the axis. The light sources may take the form of liquid crystal materials in slots in a diaphragm behind which is a separate source of illumination. The liquid crystals are used in the manner of electric shutters which are opened sequentially to permit the light beam to pass through the slots sequentially.

For the astigmatic measurement, the spherical curvature S, the degree of astigmatism C, and the angle of the astigmatic axis A can also be obtained from the image data on only two meridian lines according to the previous Japanese Patent Application No. 160083/1989 corresponding to EP-A-0,373,788 of the present applicant. In this case it is necessary that the two meridian lines are at right angles to one another. In general, if the eye to be tested is astigmatic, the ocular refracting power on a meridian line varies sinusoidally as the meridian line is rotated through 360°. Thus by measuring the ocular refracting power on two perpendicular meridian lines the astigmatic condition of the eye can be determined by basing the calculation on the sinusoidal relationship.

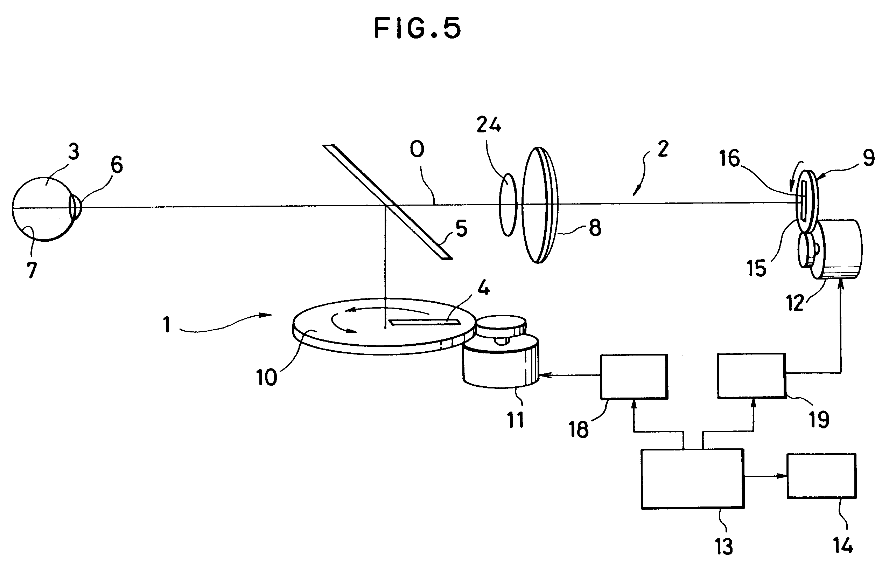

In the above embodiments, the outer portion of the reflected light beam is blocked by a light blocking member in the form of an annular plate. In the further embodiment shown in Figure 5, the light blocking member is constituted by a solid circular disc 24.

## Claims

1. Apparatus for measuring the ocular refracting power and astigmatic condition of an eye, comprising a projector system (1) including a light source (4) arranged to emit a light beam along an optical axis and to project an image of the light source onto the retina (7) of the eye (3) to be tested, a light receiving system (2) including a light blocking member (17), located at a position conjugate with the retina of a patient's eye with standard refraction, for blocking a part of the light beam reflected from the retina of the eye, a photodetector (9) which is placed at a position approximately conjugate with the pupil (6) of the eye and is responsive to the gradient of the light intensity of the light beam incident on it along a line corresponding to a meridian line on the eye and produces a signal representative of the said gradient, and a controller (13) arranged to calculate the ocular refracting power based on the said gradient on the meridian line, characterised in that the projector system (1) is so constructed that the image of the light source on the retina (7) may be rotated about the optical axis so as to allow the measurement of the refracting power on a plurality of meridian lines, the shape of the image on the retina (7) being elongate, that the light blocking member (17) is circular, and that the controller (13) is arranged to store the ocular refracting power on each of said meridian lines and to calculate the astigmatic condition of the eye (3) therefrom.

2. Apparatus as claimed in Claim 1, characterised in that the light blocking member (17) is in the form of an annular member, the edge of the aperture in which is circular.

3. Apparatus as claimed in Claim 1, characterised in that the light blocking member is in the form of a disc whose outer edge is circular.

4. Apparatus as claimed in any one of the preceding claims characterised in that the projector system (1) is so constructed that the image of the source of the retina (7) is slit-shaped.

5. Apparatus as claimed in Claim 4, characterised in that the light source (4) is slit-shaped and is connected to a first motor (11) to be rotated about the optical axis.

6. Apparatus as claimed in claim 4, characterised in that the projector system (1) includes a diaphragm in which a plurality of slit-shaped apertures are formed which may be opened and closed sequentially.

7. Apparatus as claimed in Claim 4, characterised in that the projector system (1) includes a diaphragm (22) in which a slit-shaped aperture (21) is formed and which is connected to a first motor (11) to be rotated about the optical axis.

8. Apparatus as claimed in Claim 4, 5, 6 or 7, characterised in that the photodetector (9) includes a planar light receiving element (20).

9. Apparatus as claimed in Claim 4, 5, 6 or 7, characterised in that the photodetector (9) includes a line sensor (16) which is connected to a second motor (12) to be rotated thereby.

10. Apparatus as claimed in Claims 5 and 9 or Claims 7 and 9 characterised in that the first and second motors (11, 12) are connected to respective drivers (18, 19) which are connected so that the two motors rotate in synchronism.

**Patentansprüche**

1. Gerät zur Messung der okularen Brechkraft und der astigmatischen Bedingungen eines Auges, mit einem Projektorsystem (1), das eine Lichtquelle (4) zur Emission eines Lichtstrahls längs einer optischen Achse und zum Projizieren eines Bildes der Lichtquelle auf die Retina (7) des zu untersuchenden Auges (3) aufweist, mit einem Lichtempfangssystem (2), bei dem ein an einer der Retina eines Patientenauges konjugiert zugeordneten Position gelegenes Lichtsperrelement (17) mit üblicher Brechkraft zum Blockieren eines Teils des von der Retina des Auges reflektierten Lichtstrahles vorgesehen ist, mit einem Photodektor (9), der bei einer ungefähr konjugiert zur Pupille (6) des Auges zugeordneten Position angeordnet ist und der auf den Gradienten der Lichtintensität des auf ihn auffallenden Lichtstrahls längs einer der Meridianlinie des Auges entsprechenden Linie anspricht und ein diesen Gradienten verkörperndes Signal erzeugt, und mit einem zum Berechnen der okularen Brechkraft auf Grundlage dieses Gradienten auf der Meridianlinie eingerichteten Kontroller (13), **dadurch gekennzeichnet**, daß das Projektorsystem (1) so gestaltet ist, daß das Bild der Lichtquelle auf der Retina (7) um die optische Achse so verdrehbar ist, daß die Messung der Brechkraft auf eine Vielzahl von Meridianlinien ermöglicht ist, wobei die Form des Bildes auf der Retina (7) länglich, daß das Lichtsperrelement (17) kreisförmig und daß der Kontroller (13) dafür eingerichtet ist, die okulare Brechkraft auf jeder der genannten Meridianlinien abzuspeichern und daraus die astigmatischen Bedingungen des Auges (3) zu berechnen.

2. Gerät nach Anspruch 1, dadurch gekennzeichnet, daß das Lichtsperrelement (17) die Form eines ringförmigen Elements aufweist, dessen Öffnungskante kreisförmig ist.

3. Gerät nach Anspruch 1, dadurch gekennzeichnet, daß das Lichtsperrelement in Form einer Scheibe mit kreisförmiger Außenkante ausgebildet ist.

4. Gerät nach irgendeinem der vorangehenden Ansprüche, dadurch gekennzeichnet, daß das Projektorsystem (1) so gestaltet ist, daß das Bild der Quelle der Retina (7) schlitzförmig ist.

5. Gerät nach Anspruch 4, dadurch gekennzeichnet, daß die Lichtquelle (4) schlitzförmig und mit einem ersten Motor (11) zur Rotation um die optische Achse verbunden ist.

6. Gerät nach Anspruch 4, dadurch gekennzeichnet, daß das Projektorsystem (1) eine Blendenplatte aufweist, in der eine Vielzahl von schlitzförmigen Öffnungen ausgebildet sind, die sequentiell geöffnet und geschlossen werden können.

7. Gerät nach Anspruch 4, dadurch gekennzeichnet, daß das Projektorsystem (1) eine Blendenplatte (22) aufweist, in der eine schlitzförmige Öffnung (21) ausgebildet und die mit einem ersten Motor (11) zur Rotation um die optische Achse verbunden ist.

8. Gerät nach einem der Ansprüche 4, 5, 6 oder 7, dadurch gekennzeichnet, daß der Photodetektor (9) ein flächiges Element (20) zum Lichtempfang aufweist.

9. Gerät nach Anspruch 4, 5, 6 oder 7, dadurch gekennzeichnet, daß der Photodetektor (9) einen Zeilensensor (16) aufweist, der zu seiner Rotation mit einem zweiten Motor (12) verbunden ist.

10. Gerät nach Anspruch 5 und 9 oder den Ansprüchen 7 und 9, dadurch gekennzeichnet, daß der erste (12) und der zweite Motor (11) an entsprechende Antriebe (18, 19) angeschlossen sind, die so verbunden sind, daß die beiden Motoren synchron rotieren.

**Revendications**

1. Appareil de mesure de la réfraction oculaire et de l'état d'astigmatisme d'un oeil, comprenant un système projecteur (1) incluant une source lumineuse (4) disposée de manière à émettre un faisceau lumineux le long d'un axe optique et à projeter une image de la source lumineuse sur la rétine (7) de l'oeil (3) devant être contrôlé, un système récepteur de lumière (2) comprenant un élément (17) de blocage de la lumière qui est placé en une position conjuguée avec la rétine d'un oeil de patient pour une réfraction normalisée afin de bloquer une partie du faisceau lumineux réfléchi par la rétine de l'oeil, un photodétecteur (9) qui est placé à une position approximativement conjuguée avec la pupille (6) de l'oeil et qui est sensible au gradient de l'intensité lumineuse du faisceau lumineux incident sur lui le long d'une ligne correspondant à une ligne méridienne de l'oeil et produit un signal représentatif dudit gradient, ainsi qu'un dispositif de commande (13) conçu pour calculer la réfraction oculaire sur la base dudit gradient sur la ligne méridienne, caractérisé en ce que le système projecteur (1) est construit de manière que l'image de la source lumineuse sur la rétine (7) puisse subir une rotation autour de l'axe optique de façon à permettre la mesure de la réfraction sur plusieurs lignes méridiennes, la forme de l'image sur la rétine (7) étant allongée, en ce que l'élément (17) de blocage de la lumière est circulaire et en ce que le dispositif de commande (13) est conçu pour mémoriser la réfraction oculaire sur chacune desdites lignes méridiennes et pour en calculer la condition d'astigmatisme de l'oeil.

2. Appareil selon la revendication 1, caractérisé en ce que l'élément (17) de blocage de la lumière est en forme d'un élément annulaire dont le bord du trou est circulaire.

3. Appareil selon la revendication 1, caractérisé en ce que l'élément de blocage de la lumière est en forme d'un disque dont le bord extérieur est circulaire.

4. Appareil selon l'une quelconque des revendications précédentes, caractérisé en ce que le système projecteur (1) est construit de manière que l'image de la source sur la rétine (7) aie une forme de fente.

5. Appareil selon la revendication 4, caractérisé en ce que la source lumineuse (4) est en forme de fente et en ce qu'elle est reliée à un premier moteur (11) de manière qu'elle soit entraînée en rotation autour de l'axe optique.

6. Appareil selon la revendication 4, caractérisé en ce que le système projecteur (1) comprend un diaphragme dans lequel sont réalisées plusieurs ouvertures en forme de fentes qui peuvent être ouvertes et fermées séquentiellement.

7. Appareil selon la revendication 4, caractérisé en ce que le système projecteur (1) comprend un diaphragme (22) dans lequel une ouverture (21) en forme de fente est réalisée et qui est relié à un premier moteur (11) de manière qu'il soit entraîné en rotation autour de l'axe optique.

8. Appareil selon la revendication 4, 5, 6 ou 7, caractérisé en ce que le photodétecteur (9) comprend un élément plan (20) de réception de la lumière.

9. Appareil selon la revendication 4, 5, 6 ou 7, caractérisé en ce que le photodétecteur (9) comprend un détecteur formé d'une ligne (16) et qui est relié à un second moteur (12) qui l'entraîne en rotation.

10. Appareil selon les revendications 5 et 9 ou les revendications 7 et 9, caractérisé en ce que les premier et second moteurs (11, 12) sont connectés à des circuits respectifs de commande (18, 19) qui sont connectés de manière que les deux moteurs tournent en synchronisme.

FIG.1

FIG.2

# FIG.3

EP 0 479 502 B1

# FIG.4

EP 0 479 502 B1

# FIG.5

EP 0 479 502 B1